# EUROPEAN PATENT APPLICATION

(11) **EP 4 759 317 A1**
(43) Date of publication of application: **17.06.2026**
(21) Application number: 24851732.8
(22) Date of filing: 01.08.2024
(51) Int. Cl.: A61K 31/683, A23L 33/10, A61K 31/685, A61K 35/57, A61K 35/618, A61K 35/655, A61P 21/00, A61P 43/00

(54) **MUSCLE ENHANCING AGENT, ORAL COMPOSITION FOR MUSCLE ENHANCEMENT, AND USE OF PLASMALOGEN**

(30) Priority: 09.08.2023 JP 2023129825
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO Takehiko, Fukuoka-shi, Fukuoka 813-0043 (JP); MAWATARI Shiro, Fukuoka-shi, Fukuoka 813-0016 (JP); NIWASE Shamim, Fukuoka-shi, Fukuoka 810-0054 (JP); HONSHO Masanori, Fukuoka-shi, Fukuoka 819-0395 (JP)
(74) Representative: V.O.
(86) International application number: PCT/JP2024/027474
(87) International publication number: WO 2025/033307

(57) **Abstract**

A muscle-enhancing agent includes plasmalogen as an effective component.

## Description

### Technical Field

The present disclosure relates to a muscle-enhancing agent, an oral composition for muscle enhancement, and use of plasmalogen.

### Background Art

Skeletal muscle is the largest tissue in the human body, and has important roles such as supporting of the body and moving of the limbs. When a state without use of muscle continues for a certain period due to insufficiency of exercise, bedridden conditions, cast immobilization, or the like, muscle becomes smaller, and its function declines. Human muscle decreases by 1% or more per year after the age of 40, and it decreases to about one half by the age of 80 relative to the maximum muscle mass. Loss of muscle not only threatens the period of independent and active living, that is, "healthspan", but also increases arthritis, low back pain, and chronic pain. Accordingly, in Japan, which has entered a super-aged society, problems of musculoskeletal disorders such as age-related muscle weakness (sarcopenia), in which muscle mass or muscle strength declines with aging, have become apparent.

Muscle satellite cells, which are skeletal muscle stem cells, are responsible for most of the regenerative capacity of muscle. Muscle satellite cells are usually in a resting state, and do not proliferate. However, when they are stimulated, for example, due to damage of skeletal muscle caused by strenuous exercise, they are activated to express the myogenic differentiation regulatory factor MyoD. As a result, those cells become precursor cells called myoblasts. Non Patent Literature 1 reports that decreased expression of MyoD reduces muscle mass and leads to sarcopenia.

Myoblasts differentiate into myotube cells, and the myotube cells are fused to form muscle fibers, which then constitute muscle. Muscle satellite cells are crucially responsible not only for muscle regeneration, but also for muscle growth during development and for muscle hypertrophy induced by muscle training. In the pathological conditions of intractable muscle diseases such as muscular dystrophy, and of sarcopenia, which is increasingly becoming prevalent, a decrease in the number of muscle satellite cells and functional decline of those cells are observed.

To date, methods of activation of muscle satellite cells by high-intensity exercise have been demonstrated. Non Patent Literature 2 reports that the number of muscle satellite cells and the cross-sectional area of muscle fibers increased by performing muscle training at an intensity of not less than 80% of one-repetition maximum (1RM), 8 to 12 repetitions per set for 2 to 3 sets, at a frequency of three times per week for a period of not less than 3 months.

Further, Patent Literature 1 discloses a muscle-activating agent that contains a plant extract of bergamot as an effective component, intended to activate myoblasts or promote synthesis of muscle protein.

### Citation List

### Patent Literature

Patent Literature 1: Unexamined Japanese Patent Application Publication No. 2023-029511

### Non Patent Literature

Non Patent Literature 1: Jin-A Kim and 6 others, Sinensetin regulates age-related sarcopenia in cultured primary thigh and calf muscle cells, BMC Complementary and Alternative Medicine, 2019,19:287, 1-8
Non Patent Literature 2: Lex B. Verdijk and 5 others, Satellite cell content is specifically reduced in type II skeletal muscle fibers in the elderly, Am J Physiol Endocrinol Metab, 2007, 292, E151-E157

### Summary of Invention

### Technical Problem

The high-intensity exercise disclosed in Non Patent Literature 2 imposes a considerable burden on the practitioner and can hardly be regarded as safe. High-intensity exercise is particularly difficult to perform for elderly people. The effect of the muscle-activating agent disclosed in Patent Literature 1 on muscle satellite cells is unclear. There is a demand for a highly safe muscle-enhancing agent and a highly safe oral composition for muscle enhancement that have a muscle satellite cell-activating action.

The present disclosure has been made in view of the above circumstances and aims to provide a highly safe muscle-enhancing agent and a highly safe oral composition for muscle enhancement that have a muscle satellite cell-activating action. The present disclosure also aims to provide use of plasmalogen for production of the muscle-enhancing agent or the oral composition for muscle enhancement.

### Solution to Problem

A muscle-enhancing agent according to a first aspect of the present disclosure includes plasmalogen as an effective component.

An oral composition for muscle enhancement according to a second aspect of the present disclosure includes plasmalogen as an effective component.

The plasmalogen may be plasmalogen extracted from an animal tissue.

The animal tissue may be an animal tissue selected from the group consisting of shellfish, sea squirts, and birds.

The animal tissue may be a tissue of a scallop.

Use according to a third aspect of the present disclosure is use of plasmalogen for production of a muscle-enhancing agent or an oral composition for muscle enhancement.

### Advantageous Effects of Invention

According to the present disclosure, a highly safe muscle-enhancing agent and a highly safe oral composition for muscle enhancement that have a muscle satellite cell-activating action are provided. Further, according to the present disclosure, use of plasmalogen for production of the muscle-enhancing agent or the oral composition for muscle enhancement is also provided.

### Brief Description of Drawings

FIG. 1 is a diagram illustrating fluorescence microscopy images of immunostained muscle fibers of thigh muscle;
FIG. 2A is a diagram illustrating a result of Western blotting for phosphorylated AMPK (pAMPK), as an analysis result of phosphorylation of AMPK in the soleus muscle;
FIG. 2B is a diagram illustrating a relative amount of pAMPK/AMPK based on the Western blotting result illustrated in FIG. 2A;
FIG. 3A is a diagram illustrating a Western blotting result for MyoD, as an evaluation result of expression of MyoD in soleus muscle;
FIG. 3B is a diagram illustrating a relative amount of MyoD based on the Western blotting result illustrated in FIG. 3A;
FIG. 4A is a diagram illustrating bright-field images of thigh muscle; and
FIG. 4B is a diagram illustrating the muscle fiber diameter.

### Description of Embodiments

Embodiments according to the present disclosure are described below with reference to drawings. In this regard, the present disclosure is not limited by the below-described embodiments and the drawings. Note that, in the below-described embodiments, the expression, "have", "include", or "contain" encompasses the meaning of "composed of" or "be constituted by".

A muscle-enhancing agent according to the present embodiment includes plasmalogen as an effective component. Plasmalogen belongs to a subclass specific to glycerophospholipids characterized by the presence of a vinyl ether bond at the sn-1 position and an ester bond at the sn-2 position of the glycerol backbone. Plasmalogen is present at high concentration in the cell membrane of a large number of mammalian tissues.

The plasmalogen included in the muscle-enhancing agent is not limited as long as it is generally classified as plasmalogen. Examples of the plasmalogen include Pls, PlsCho, inositol-type plasmalogen, and serine-type plasmalogen. Pls has a structure in which -CH₂CH₂NH₂ is bound to the oxygen atom that is bound to phosphorus at the sn-3 position of the glycerol backbone. PlsCho has a structure in which -CH₂CH₂N(CH₃)₃ is bound to the oxygen atom that is bound to phosphorus at the sn-3 position of the glycerol backbone.

Preferably, the plasmalogen is extracted from an animal tissue. The animal tissue is not particularly limited as long as it is an animal tissue containing plasmalogen. Examples of the animal tissue include tissues of animals selected from the group consisting of shellfish, sea squirts, and birds. More specifically, tissues of aquatic animals such as shellfish, sea squirts, sea cucumbers, salmon, Pacific saury, and skipjack tuna are preferred as the animal tissue. Preferably, the animal tissue is a tissue of shellfish. An edible part of the animal tissue is preferred. The animal tissue may be a cut tissue, or may be a tissue that has been pulverized to extract plasmalogen more efficiently.

Examples of the shellfish include bivalves or snails, such as scallops, mussels, and abalones. Scallops are particularly preferred as the shellfish. Scallops are edible bivalves belonging to the family Pectinidae, and their examples include scallops belonging to the genus *Mizuhopecten* or the genus *Pecten.* Specific examples of the shellfish include scallops harvested in Japan (*Mizuhopecten yessoensis*) and European scallops harvested in Europe (*Pecten maximus* (Linnaeus)). Examples of edible parts of the scallops include adductor muscle and fringe.

Sea squirts are edible chordates belonging to the family Pyuridae, and their examples include those belonging to the genus *Halocynthia* or *Halocynthia aurantium.* Specific examples of the sea squirts include maboya (*Halocynthia roretzi*) and akaboya (*Halocynthia aurantium*). Examples of edible parts of the sea squirts include the flesh portion (fascial body).

Examples of the birds include edible birds. Examples of the birds include chickens, Silkies, and ducks. As the bird tissue, breast meat is preferred because it abundantly contains plasmalogen.

Plasmalogen can be extracted using an organic solvent or an aqueous organic solvent, and is preferably extracted using enzyme treatment in combination. Examples of the extraction method for plasmalogen include an ethanol extraction method and a hexane extraction method.

In the ethanol extraction method, the animal tissue may be exposed to an extraction liquid containing ethanol. The extraction liquid containing ethanol used in the extraction step may be aqueous ethanol or a mixture of ethanol and another organic solvent. The ethanol concentration in the extraction liquid is not less than 50% by mass, preferably not less than 80% by mass, more preferably not less than 95% by mass, or may be 100% by mass. The amount of the ethanol used is, for example, preferably 100 to 3000 mL, more preferably 200 to 2000 mL, still more preferably 250 to 1000 mL per 100 g of the animal tissue. The extraction using ethanol may be carried out a plurality of times.

Preferably, the animal tissue exposed to the extraction liquid containing ethanol is an animal tissue treated with a protease. The protease is, for example, a protease derived from a filamentous fungus of the genus *Aspergillus,* the genus *Rhizopus,* or the like; a protease derived from a bacterium of the genus *Bacillus* or the like; or a protease extracted from a plant such as papaya or pineapple. As the protease, a commercially available protease may be used. In particular, a protease derived from a filamentous fungus is preferred, and a protease derived from koji mold is more preferred. Since plasmalogen is unstable under acidic and basic conditions, a neutral protease is preferred.

The amount of the protease used is, for example, 0.1 to 10.0 g, preferably 0.2 to 8.0 g, more preferably 0.3 to 5.0 g for 100 g of the animal tissue.

After the extraction, solids are removed, and the extraction liquid is collected, followed, when necessary, by performing solidification by drying, to obtain an extract containing plasmalogen. The plasmalogen may be concentrated by subjecting the extract to concentration treatment.

In the case of a hexane extraction method, total lipids contained in an extract obtained by an ethanol extraction method are subjected to extraction treatment with a mixed solvent of n-hexane and a water-soluble ketone solvent, to achieve separation into an insoluble fraction and a soluble fraction. The water-soluble ketone solvent is, for example, acetone, methyl ethyl ketone, or a combination thereof. The water-soluble ketone solvent is preferably acetone. In cases where a mixture of n-hexane and acetone is used as the mixed solvent, their volume ratio is, for example, 4:6 to 6:4, preferably 4.5:5.5 to 5.5:4.5. The amount of the mixed solvent used is, for example, 10 to 30 mL per 1 g (dry mass) of total lipids.

After drying the soluble fraction obtained by the extraction treatment with the mixed solvent, plasmalogen can be separated and recovered by performing extraction treatment with a water-soluble ketone solvent.

The content of plasmalogen in the muscle-enhancing agent according to the present embodiment is adjusted as appropriate. For example, the muscle-enhancing agent may contain, as an effective component, 0.000001 to 99.9% by mass, 0.00001 to 99.8% by mass, 0.0001 to 99.7% by mass, 0.001 to 99.6% by mass, 0.01 to 99.5% by mass, 0.1 to 99% by mass, 0.5 to 60% by mass, 1 to 50% by mass, or 1 to 2% by mass plasmalogen.

Examples of the subject to which the muscle-enhancing agent according to the present embodiment is administered include cells, biological tissues, organisms, and animals. When the muscle-enhancing agent is used as a pharmaceutical, it is administered to a human or an animal. The animal is preferably a mammal, more specifically, a dog, cat, cow, pig, horse, sheep, deer, or the like.

The route of administration of the muscle-enhancing agent according to the present embodiment to a human or the like is not particularly limited. The muscle-enhancing agent is preferably used as a topical preparation, an injection, or an oral preparation. The muscle-enhancing agent may contain, for example, plasmalogen and a pharmacologically acceptable carrier. The pharmacologically acceptable carrier includes various organic carrier substances or inorganic carrier substances used as formulation materials. Examples of the pharmacologically acceptable carrier include, for solid preparations, excipients, binders, disintegrants, lubricants, stabilizers, taste- and odor-correcting agents, and the like; and, for liquid preparations, solvents, solubilizing agents, suspending agents, isotonic agents, buffers, taste- and odor-correcting agents, and the like. Further, an additive such as an antiseptic agent, an antioxidant, a coloring agent, or a sweetener may be added, when necessary.

Examples of the excipients include lactose, white sugar, D-mannitol, D-sorbitol, starch, pregelatinized starch, dextrin, crystalline cellulose, low-substituted hydroxypropyl cellulose, sodium carboxymethyl cellulose, gum arabic, pullulan, light anhydrous silicic acid, synthetic aluminum silicate, magnesium aluminometasilicate, xylitol, sorbitol, and erythritol.

Examples of the binders include the above excipients, and also include pregelatinized starch, sucrose, gelatin, gum arabic, methyl cellulose, carboxymethyl cellulose, sodium carboxymethyl cellulose, crystalline cellulose, white sugar, D-mannitol, trehalose, dextrin, pullulan, hydroxypropyl cellulose, hydroxypropyl methylcellulose, and polyvinylpyrrolidone.

Examples of the disintegrants include the above excipients, and also include lactose, white sugar, starch, carboxymethyl cellulose, calcium carboxymethyl cellulose, croscarmellose sodium, sodium carboxymethyl starch, low-substituted hydroxypropyl cellulose, light anhydrous silicic acid, and calcium carbonate.

Examples of the lubricants include magnesium stearate, calcium stearate, talc, colloidal silica, and polyethylene glycol.

Examples of the stabilizers include *p*-hydroxybenzoic acid esters such as methylparaben and propylparaben; alcohols such as chlorobutanol, benzyl alcohol, and phenylethyl alcohol; benzalkonium chloride; acetic anhydride; and sorbic acid. Examples of the taste- and odor-correcting agents include sweeteners, acidulants, and flavoring agents.

Examples of the solvents include water for injection, physiological saline, Ringer's solution, alcohols, propylene glycol, polyethylene glycol, sesame oil, corn oil, olive oil, and cottonseed oil. Examples of the solubilizing agents include polyethylene glycol, propylene glycol, D-mannitol, trehalose, benzyl benzoate, ethanol, tris(hydroxymethyl)aminomethane, cholesterol, triethanolamine, sodium carbonate, sodium citrate, sodium salicylate, and sodium acetate.

Examples of the suspending agents include surfactants such as stearyl triethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, sodium carboxymethyl cellulose, methyl cellulose, hydroxymethyl cellulose, hydroxyethyl cellulose, and hydroxypropyl cellulose; polysorbates; and polyoxyethylene hydrogenated castor oil.

Examples of the isotonic agents include sodium chloride, glycerin, D-mannitol, D-sorbitol, glucose, xylitol, and fructose. Examples of the buffers include buffer solutions of phosphate, acetate, carbonate, and citrate.

Examples of the preservatives include paraoxybenzoic acid esters, chlorobutanol, benzyl alcohol, phenethyl alcohol, dehydroacetic acid, and sorbic acid. Examples of the antioxidants include sulfite and ascorbate. Examples of the coloring agents include water-soluble tar dyes, lake pigments, and natural colorants. Examples of the sweeteners include saccharin sodium, dipotassium glycyrrhizinate, aspartame, and stevia.

The muscle-enhancing agent according to the present embodiment is produced by a known method, and provided in the form of liquids, creams, ointments, tablets, granules, fine granules, powders, tablets, capsules, or the like.

The dose of the muscle-enhancing agent according to the present embodiment is appropriately determined in accordance with the age, body weight, symptoms, and the like of the human or animal to whom the agent is to be administered. The muscle-enhancing agent is administered such that the plasmalogen is in an effective amount. The effective amount is an amount of plasmalogen required to obtain a desired result, that is, an amount required to delay, inhibit, prevent, reverse, or cure the progression of the condition to be treated or managed. Examples of a condition or disease treated and prevented by muscle enhancement include musculoskeletal disorders such as sarcopenia, and intractable muscle diseases such as muscular dystrophy.

The dose, per day for an adult, of the muscle-enhancing agent according to the present embodiment is, for example, not less than 0.000001 µg, preferably not less than 1 µg or not less than 500 µg, more preferably not less than 1000 µg. The upper limit of the dose is, for example, 20,000 µg per day, preferably 10,000 µg per day. The muscle-enhancing agent may be administered once or a plurality of times per day. The muscle-enhancing agent may also be administered at various dosing frequencies, for example, every other day, once a week, every other week, or once a month. When necessary, a dose outside the above-described range can also be used.

As shown in the Examples below, plasmalogen as the effective component in the muscle-enhancing agent according to the present embodiment has the effect of activating muscle satellite cells and thickening muscle fibers. The muscle-enhancing agent is useful for muscle repair and strengthening, and for increasing muscle mass and muscle strength. Since plasmalogen is a substance extracted from an animal tissue or the like, the muscle-enhancing agent according to the present embodiment is highly safe.

The muscle-enhancing agent according to the present embodiment may be used as a reagent for the purpose of enhancing muscle in in vitro and in vivo experiments.

Another aspect of the present embodiment provides an oral composition for muscle enhancement. Specific examples of the oral composition include supplements, food and beverage compositions, functional foods, and food additives.

The forms of the supplements are not limited, and may be any of tablets, powders, granules, capsules, sugar-coated tablets, film formulations, troches, chewable formulations, solutions, emulsions, and suspensions. The supplements may include any component that is normally used for supplements.

"Functional food" means a food or beverage consumed for the purpose of maintaining health, and examples of the functional food include: foods for specified health uses, foods with function claims, and foods with nutrient function claims, which are foods with health claims; health foods; and dietary supplements. The functional foods are preferably foods for specified health uses or foods with nutrient function claims, which are foods with health claims. In cases of commercialization as a functional food, the oral composition for muscle enhancement may include various additives for use in foods, such as colorants, preservatives, thickening stabilizers, antioxidants, bleaching agents, antibacterial and antifungal agents, acidulants, sweeteners, seasonings, emulsifiers, fortifiers, agents for production, and flavors.

The functional food may be either a food or beverage, and is not limited as long as the functional food can be orally taken. Examples of the forms of the functional food include beverages, confectionery, processed cereal products, kneaded products, dairy products, and seasonings. Examples of the beverages include nutritional drinks, soft drinks, black tea, and green tea. Examples of the confectionery include candies, cookies, tablets, chewing gums, and jellies. Examples of the processed cereal products include noodles, bread, cooked rice, and biscuits. Examples of the kneaded products include sausage, ham, and kamaboko (boiled fish paste). Examples of the dairy products include butter and yogurt.

The oral composition for muscle enhancement may be added to a food as a food additive. In such a case, the food additive may be a paste, a gel-like agent, a powder, a liquid, a suspension, an emulsion, a granule, or the like from the viewpoint of easily adding the food additive to foods.

The oral composition for muscle enhancement according to the present embodiment may contain, to an extent that maintains effectiveness related to the muscle enhancement, water, vitamins, minerals, organic acids, organic bases, fruit juice, flavors, functional components, food additives, and the like. The oral composition for muscle enhancement may be produced by a known method in which components other than plasmalogen are added when necessary.

The above oral composition for muscle enhancement may be contained dividedly in one or more containers such that the daily amount of intake equals the above-described amount of intake. In this case, each container preferably contains a one-day portion of the oral composition for muscle enhancement.

From the viewpoint of the fact that the oral composition for muscle enhancement contains plasmalogen and is used for muscle enhancement, it is provided in the form of a product that can be distinguished from other products. For example, at least one of the packaging, instructions, and advertisements for the product relating to the oral composition for muscle enhancement has a label indicating that the composition has a muscle-enhancing function.

In cases where the oral composition for muscle enhancement according to the present embodiment is provided as a food or beverage composition, the composition may be provided or made commercially available as a food or beverage with a label indicating uses (including health uses) for muscle enhancement, and also for prevention of musculoskeletal disorders and intractable muscle diseases, reduction of the risk of these diseases, alleviation of symptoms of the diseases, treatment of the diseases, and the like. The act of "indication" includes all acts that inform consumers of the aforementioned application. Any expression that evokes or suggests the aforementioned application is considered an act of "indication", regardless of the purpose, content, object, medium, and the like of the indication.

The "indication" is preferably made in a manner that allows the consumers to directly recognize the aforementioned application. Specifically, this includes acts such as an act of transferring, delivering, exhibiting for transfer or delivery, or importing food and beverage products or product packaging with the aforementioned application described; an act of exhibiting or distributing advertisements, price lists, or transaction documents relating to the products with the aforementioned application described, or providing information of the content with the aforementioned application described via electromagnetic means such as the Internet.

The "indication" may include indications as health foods, functional foods, enteral nutritional foods, foods for special uses, foods with health claims, foods for specified health uses, foods with nutrient function claims, foods with function claims, quasi-drugs, and the like. Among these, particularly, indications approved under systems for foods for specified health uses, foods with nutrient function claims, or foods with function claims, or similar systems are included. Specifically, examples of the indications include an indication as foods for specified health uses, an indication as conditional foods for specified health uses, an indication of effects on body structure or function, an indication for disease risk reduction, and an indication of functionality based on scientific evidence. More specifically, typical examples thereof include an indication as foods for specified health uses, in particular, an indication of health purposes, and similar indications.

In another aspect of the present embodiment, use of plasmalogen for production of a muscle-enhancing agent or an oral composition for muscle enhancement is provided. Another aspect of the present embodiment provides a method of muscle enhancement including a step of administering plasmalogen to a subject. Another aspect of the present embodiment provides plasmalogen for use in muscle enhancement. The muscle enhancement includes prevention, suppression, inhibition, and recovery of a decrease in muscle mass or muscle strength.

The present disclosure is described more concretely by the following Examples. However, the present disclosure is not limited by the Examples.

### Examples

### [Preparation of sPls]

sPls was prepared from a scallop (*Mizuhopecten yessoensis*) by the following method. Kokulase P (Mitsubishi-Chemical Foods Corporation) was added to, and mixed with, fresh scallop mantle. Ethanol was added to the resulting mixture, and the mixture was subjected to suction filtration. The resulting filtrate was dried using a rotary evaporator, to obtain sPls.

### [Administration of sPls]

sPls was administered, in drinking water, at a dose of 1 mg/kg to 4-week-old male c57BL6J mice (four mice per group, n = 4) for 4 weeks. Soleus muscle and thigh muscle were collected from each mouse and analyzed as follows.

### [Evaluation of AMPK activation by immunostaining]

The thigh muscle was fixed with 4% paraformaldehyde (PFA) solution, and rinsed with dH₂O. The muscle was then reacted with 0.2% collagenase I solution at 37°C for 3 hours to obtain a sample. The 0.2% collagenase I solution contained collagenase type I (C0130, manufactured by Sigma-Aldrich) at a concentration of 0.2% in a buffer (50 mM Tris-HCl, 0.36 mM CaCl₂, pH 7.4).

The sample was washed with phosphate-buffered saline (PBS) and rinsed with dH₂O. Muscle fibers were then loosened under a stereomicroscope. Single muscle fibers were placed on a slide glass, dried at 65°C, and then dried at 37°C for 18 hours.

The sample was dried at 65°C for 15 minutes, and then fixed with 4% PFA solution for 40 minutes at room temperature. The sample was washed with PBS, and treated with 0.5% Tx-100/PBS at room temperature for 6 minutes. The sample was washed with PBS, and then treated with a blocking buffer (1% BSA-PBS) at room temperature for 20 minutes.

Each of a phospho-AMPKα (Thr172) antibody (#2535, manufactured by Cell Signaling technology) and a MyoD antibody (NB120-788, manufactured by Novus Biologicals) was diluted 2000-fold with 5%BSA-PBS, and used as a primary antibody. The primary antibody was reacted with the sample at 4°C for 18 hours. The sample was washed with PBS, and reacted, at room temperature for 1 hour in the dark, with a solution prepared by diluting Alexa Fluor 488 anti-rabbit IgG, Alexa Fluor 568 anti-mouse IgG (both manufactured by Invitrogen), and 3 mM DAPI 2000-fold in 1% BSA-PBS. The sample was washed with PBS and rinsed with dH₂O, followed by embedding with a cover glass and observation with BZ-X810 (manufactured by Keyence).

### [Analysis of AMPK phosphorylation and MyoD expression]

Soleus muscle was disrupted by homogenization in Buffer A (0.25 M sucrose, 10 mM Hepes-KOH (pH 7.5), 1 mM EDTA, protease inhibitor cocktail) using injection needles from 18G to 25G. After protein quantification, aliquots of the same amount of protein were subjected to electrophoresis. Subsequently, the protein was transferred onto a PVDF membrane, and detected by Western blotting using 2000-fold diluted antibody solutions of a phospho-AMPKα (Thr172) antibody (#2535, manufactured by Cell Signaling technology) and an AMPKα antibody (#5831, manufactured by Cell Signaling technology). Signals were quantified using Multi Gauge software version 3.0 software (manufactured by FUJIFILM Corporation). The signal obtained with the phospho-AMPKα (Thr172) antibody was divided by the signal obtained with the AMPK antibody, to perform normalization. Further, by taking the value obtained from untreated cells as 1, the signal intensity in the cells subjected to each treatment was expressed as a relative value. The operation was carried out three or more times, and the results were expressed as the mean and standard deviation.

Samples prepared from soleus muscle in the same manner were subjected to detection and quantification with a MyoD antibody (NB120-788, manufactured by Novus Biologicals, 2000-fold dilution) and a GAPDH antibody (M171-3, manufactured by MBL, 4000-fold dilution).

### [Measurement of muscle fiber diameter]

Single muscle fibers prepared as described above from thigh muscle were brought into focus at the depth at which the fibers appeared thickest, and their bright-field images were captured with BZ-X810 (manufactured by Keyence). Subsequently, the diameter was measured at two or three positions of each muscle fiber. The measurement was carried out at a total of 9 positions for muscle fibers without sPls administration, and at a total of 29 positions for muscle fibers with sPls administration.

### [Results]

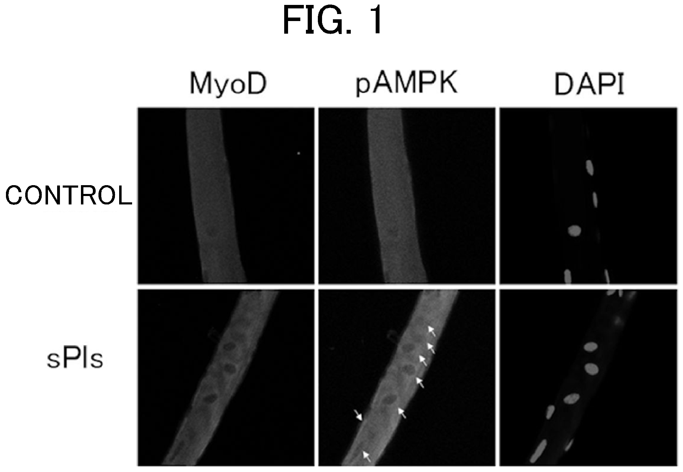

FIG. 1 illustrates the results of immunostaining for the evaluation of AMPK activation. sPls increased the expression of pAMPK, and the expression of MyoD, which is a marker of muscle differentiation, in individual cells in the thigh muscle. This indicates that sPls increased muscle satellite cells.

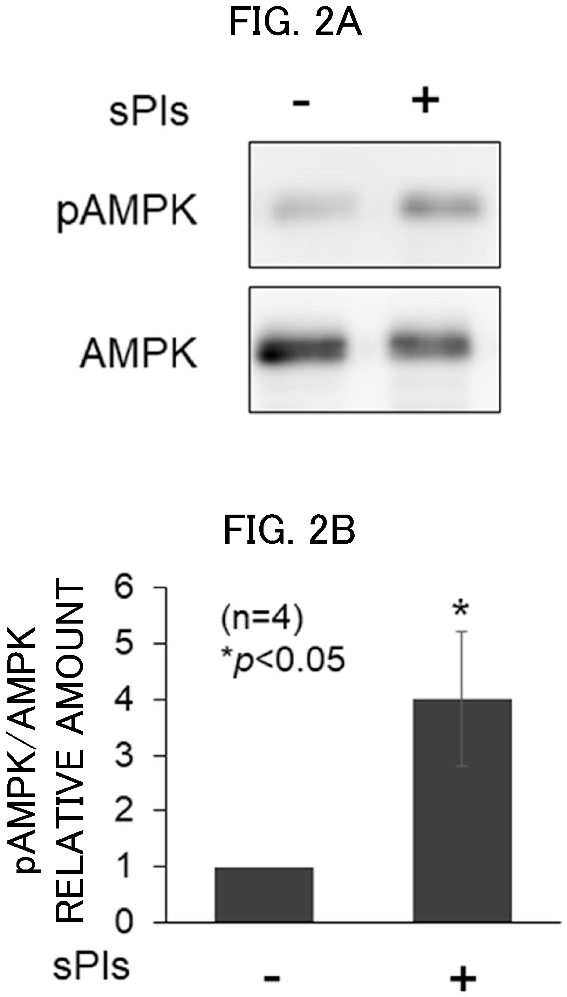

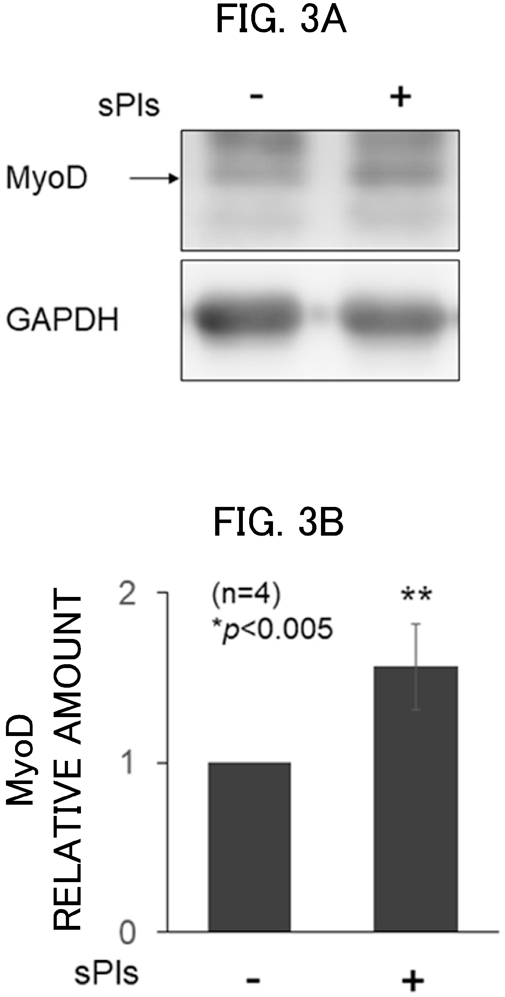

FIG. 2A illustrates the Western blotting result for pAMPK. FIG. 2B illustrates the relative amount of pAMPK/AMPK based on the Western blotting result. Activation of AMPK by sPls was found in the soleus muscle. FIG. 3A illustrates the Western blotting result for MyoD. FIG. 3B illustrates the relative amount of MyoD based on the Western blotting result. An increase in MyoD by sPls was found in the soleus muscle.

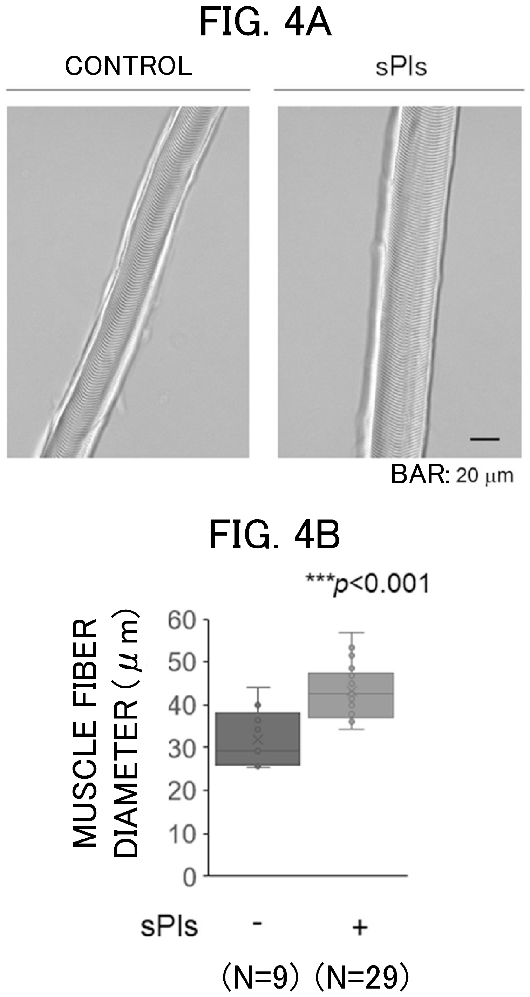

FIG. 4A illustrates bright-field images of the thigh muscle. FIG. 4B illustrates the diameters of muscle fibers. sPls was found to activate APMK in the muscle, resulting in thickening of muscle fibers.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-129825, filed on August 9, 2023, the entire disclosure of which is incorporated by reference herein.

### Industrial Applicability

The present disclosure is useful for a muscle-enhancing agent.

## Claims

1. A muscle-enhancing agent comprising plasmalogen as an effective component.

2. An oral composition for muscle enhancement, comprising plasmalogen as an effective component.

3. The muscle-enhancing agent according to claim 1 or the oral composition for muscle enhancement according to claim 2, wherein the plasmalogen is plasmalogen extracted from an animal tissue.

4. The muscle-enhancing agent or the oral composition for muscle enhancement according to claim 3, wherein the animal tissue is an animal tissue selected from a group consisting of shellfish, sea squirts, and birds.

5. The muscle-enhancing agent or the oral composition for muscle enhancement according to claim 3, wherein the animal tissue is a tissue of a scallop.

6. Use of plasmalogen for production of a muscle-enhancing agent or an oral composition for muscle enhancement.
